# DEMANDE DE BREVET EUROPEEN

(11) **EP 2 380 520 A1**
(43) Date de publication de la demande: **26.10.2011**
(21) Numéro de dépôt: 11163594.2
(22) Date de dépôt: 22.04.2011
(51) Int. Cl.: A61B 18/14

(54) **Pince électro-chirurgicale et procédé d'utilisation d'une telle pince**

(30) Priorité: 23.04.2010 FR 1001748
(71) Demandeur: Noury, Guillaume, 78670 Villennes sur Seine (FR)
(72) Inventeur: Noury, Guillaume, 78670 Villennes sur Seine (FR)
(74) Mandataire: CAPRI

(57) **Abrégé**

Pince électro-chirurgicale (10), comprenant deux branches (11, 12) dont chacune est pourvue d'un mors (13, 14) et d'une connexion électrique (110, 120) pour alimenter du courant électrique auxdits mors (13, 14), lesdites branches (11, 12) étant articulées autour d'un axe (15) entre une position ouverte et une position fermée, lesdits mors (13, 14) comportant des extrémités libres (130, 140) configurées de telle sorte qu'en position fermée des mors (13, 14), lesdites extrémités (130, 140) sont en contact l'une de l'autre, formant un court-circuit en cas d'alimentation électrique dans cette position fermée, ledit court-circuit en position fermée formant un arc électrique entre lesdits mors (13, 14), ladite pince comportant des moyens de découpe de tissus thermosoudés, lesdits moyens de découpe étant formés par ledit arc électrique.

## Description

La présente invention concerne une pince électro-chirurgicale et un procédé de thermoscission d'une cible.

Les pinces électro-chirurgicales sont, comme leur nom l'indique, principalement utilisées en chirurgie, notamment pour souder thermiquement des vaisseaux du corps. Elles comportent généralement deux branches, similaires à des ciseaux, que le chirurgien peut amener en position de fermeture, afin de refermer les mors de la pince autour d'une cible. La pince est alimentée par un courant électrique qui permet en position de fermeture des mors, de chauffer et de transformer les tissus maintenus entre les mors fermés, pour former une soudure par thermofusion. Lorsque les deux mors de la pince ont des polarités différentes, la pince est dite bipolaire. Un inconvénient des pinces existantes est qu'elles ne permettent généralement pas la découpe du vaisseau soudé. Après soudure, le chirurgien doit alors retirer la pince et la remplacer par un dispositif de coupe, ce qui complique l'opération. En variante, il existe des pinces coupantes, pourvues d'une lame pour découper le vaisseau après soudure. Ce type de pince est toutefois très complexe et donc cher à fabriquer. L'utilisation de la pince est également plus compliquée, le chirurgien devant d'abord souder le vaisseau avant d'actionner le système de coupe intégré, au moyen d'un actionneur distinct. Les documents US 5 827 281, WO 99/23933, WO 95/02366, US 2002/120267 et US 2002/123667 décrivent des dispositifs de l'art antérieur qui cherchent à éviter les courts-circuits.

La présente invention a pour but de fournir une pince électro-chirurgicale et un procédé d'utilisation de celle-ci qui ne reproduisent pas les inconvénients susmentionnés.

En particulier, la présente invention a pour but de fournir une pince électro-chirurgicale qui permette de manière simple de réaliser à la fois la thermosoudure et la découpe d'un vaisseau cible.

La présente invention a aussi pour but de fournir une pince électro-chirurgicale qui soit simple et peu coûteuse à fabriquer, à assembler et à utiliser.

La présente invention a donc pour objet une pince électro-chirurgicale, comprenant deux branches dont chacune est pourvue d'un mors et d'une connexion électrique pour alimenter du courant électrique auxdits, lesdites branches étant articulées autour d'un axe entre une position ouverte et une position fermée, lesdits mors comportant des extrémités libres configurées de telle sorte qu'en position fermée des mors, lesdites extrémités sont en contact l'une de l'autre, formant un court-circuit en cas d'alimentation électrique dans cette position fermée, ledit court-circuit en position fermée formant un arc électrique entre lesdits mors, ladite pince comportant des moyens de découpe de tissus thermosoudés, lesdits moyens de découpe étant formés par ledit arc électrique.

Avantageusement, ladite pince est du type bipolaire.

La présente invention a aussi pour objet un procédé d'utilisation d'une telle pince electro-chirurgicale, comportant les étapes de fournir une pince électro-chirurgicale telle que décrite ci-dessus, disposer lesdits mors ouverts de ladite pince autour d'une cible, tel qu'un tissu à thermosouder, alimenter du courant électrique dans lesdits mors en les refermant progressivement autour de ladite cible pour former une soudure par thermofusion de ladite cible, fermer complètement lesdits mors pour mettre en contact les extrémités libres desdits mors, alimenter du courant électrique auxdits mors dans cette position fermée pour créer un arc électrique entre lesdits mors fermés, et retirer la pince en passant ledit arc électrique sur ladite thermosoudure pour la découper.

Ces caractéristiques et avantages et d'autres de la présente invention apparaîtront plus clairement au cours de la description détaillée suivante d'un mode de réalisation avantageux de celle-ci, faite en référence aux dessins joints, donnés à titre d'exemples non limitatifs, et sur lesquels :
- la figure 1 est une vue schématique en perspective d'une pince électro-chirurgicale selon un mode de réalisation avantageux de l'invention,
- la figure 2 est une vue de détail des mors de la pince de la figure 1, avant thermosoudure d'une cible,
- la figure 3 est une vue similaire à celle de la figure 2, après thermosoudure, et
- la figure 4 est une vue similaire à celles des figures 2 et 3, illustrant l'étape de scission après thermosoudure.

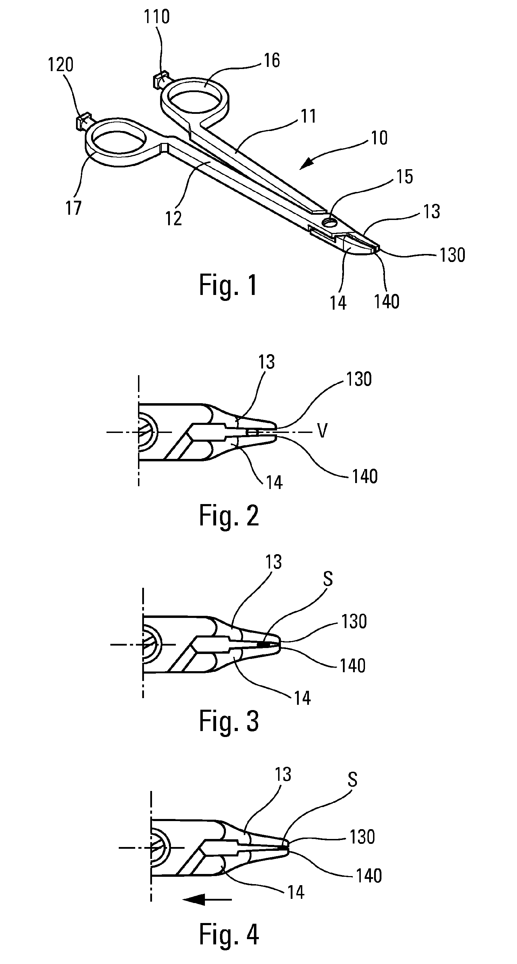

En référence à la figure 1, il est représenté un exemple de pince chirurgicale utilisable avec la présente invention. Il est entendu que toutes pinces électro-chirurgicales, même de formes différentes, pourraient être utilisées. La pince 10 comporte de manière classique deux branches 11,12, chacune pourvue d'un mors 13, 14, les branches étant articulées autour d'un axe 15, à la manière d'une paire de ciseaux, entre une position ouverte et une position fermée. Des moyens de manipulation 16, 17, typiquement des oeillets pour les doigts, sont prévus pour permettre au chirurgien d'actionner la pince.

La pince 10 est une pince électro-chirurgicale, elle est donc alimentée en courant électrique. A cet égard, chaque branche 11, 12 de la pince comporte une connexion électrique 110, 120 permettant le branchement d'une alimentation électrique (non représentée). Ces connexions électriques 110, 120 peuvent être quelconques, et le câble d'alimentation (non représenté) peut être branché d'une manière quelconque.

De manière classique, la soudure par thermofusion ou thermosoudure d'une cible est réalisée en plaçant les mors 13, 14 ouverts de la pince autour d'un cible V, typiquement un tissu à thermosouder, tel qu'un vaisseau sanguin. Le chirurgien referme alors les mors autour de ladite cible tout en alimentant du courant électrique auxdits mors. Ce courant passe alors dans les tissus de la cible pincée par les mors. Ceci provoque la soudure par thermofusion de ladite cible V, formant ainsi une soudure S.

Comme visible sur les figures 2 et 3, la cible V a une certaine épaisseur qui empêche la fermeture immédiate de la pince. Ce n'est que lorsque la soudure S est réalisée, dont l'épaisseur est nettement inférieure à celle de la cible, que la pince peut être complètement fermée.

Les pinces électro-chirurgicales existantes, telles que celles décrites notamment dans les documents US 5 827 281, WO 99/23933 et WO 95/02366, sont réalisées de telle sorte qu'à aucun moment, même en position fermée, les mors ne se touchent, pour éviter les courts-circuits.

De manière très surprenante, il a été constaté qu'en configurant les extrémités libres 130, 140 des mors 13, 14 de telle sorte qu'en position fermée ils se touchent, le court-circuit qui se crée lors d'une alimentation électrique dans cette position fermée forme un arc électrique entre les mors, qui peut être utilisé pour découper la soudure S. Ainsi, si à ce moment, le chirurgien déplace la pince fermée sur la soudure S, celle-ci sera découpée par ledit arc électrique. Le fait de prévoir le contact au niveau des extrémités libres permet ainsi au chirurgien de simplement retirer la pince fermée et alimentée en courant en passant l'arc électrique créé par le court-circuit sur la soudure S pour la découper.

L'invention permet donc de réaliser la thermosoudure et la découpe avec un dispositif très simplifié, ne nécessitant notamment pas de dispositif de coupe à lame. De même, la manipulation par le chirurgien est également simplifiée. Il ne doit plus ressortir la pince pour la remplacer par un dispositif de coupe distinct, et il n'a plus besoin d'actionner séparément un dispositif de coupe qui serait intégré à la pince. Au contraire, il lui suffit de prolonger son mouvement exercé pour réaliser la soudure, et de retirer la pince fermée en passant sur la soudure pour la découper.

L'invention va clairement à l'encontre d'un préjugé dans le domaine technique des pinces électro-chirurgicales en exploitant un court-circuit qui jusqu'à présent avait toujours été considéré comme non souhaitable dans ce type de dispositifs.

Diverses modifications peuvent être apportées par l'homme de l'art, sans sortir du cadre de la présente invention tel que défini par les revendications annexées.

## Revendications

1. Pince électro-chirurgicale (10), comprenant deux branches (11, 12) dont chacune est pourvue d'un mors (13, 14) et d'une connexion électrique (110, 120) pour alimenter du courant électrique auxdits mors (13, 14), lesdites branches (11, 12) étant articulées autour d'un axe (15) entre une position ouverte et une position fermée, **caractérisée en ce que** lesdits mors (13, 14) comportent des extrémités libres (130, 140) configurées de telle sorte qu'en position fermée des mors (13, 14), lesdites extrémités (130, 140) sont en contact l'une de l'autre, formant un court-circuit en cas d'alimentation électrique dans cette position fermée, ledit court-circuit en position fermée formant un arc électrique entre lesdits mors (13, 14), ladite pince comportant des moyens de découpe de tissus thermosoudés, lesdits moyens de découpe étant formés par ledit arc électrique.

2. Pince selon la revendication 1, dans laquelle ladite pince est du type bipolaire.

3. Procédé d'utilisation d'une pince electro-chirurgicale selon l'une quelconque des revendications précédentes, **caractérisé en ce qu'**il comporte les étapes de fournir une pince électro-chirurgicale (10), disposer lesdits mors ouverts (13, 14) de ladite pince autour d'une cible (V), tel qu'un tissu à thermosouder, alimenter du courant électrique dans lesdits mors en les refermant progressivement autour de ladite cible pour former une soudure par thermofusion (S) de ladite cible, fermer complètement lesdits mors pour mettre en contact les extrémités libres (130, 140) desdits mors, alimenter du courant électrique auxdits mors dans cette position fermée pour créer un arc électrique entre lesdits mors fermés, et retirer la pince en passant ledit arc électrique sur ladite thermosoudure pour la découper.
